# EUROPEAN PATENT APPLICATION

(11) **EP 4 600 235 A1**
(43) Date of publication of application: **13.08.2025**
(21) Application number: 23874533.5
(22) Date of filing: 15.08.2023
(51) Int. Cl.: C07C 1/12, C07C 9/04, B01J 8/02

(54) **GENERATION DEVICE AND GENERATION METHOD**

(30) Priority: 07.10.2022 JP 2022162809
(71) Applicant: Kanadevia Corporation, Osaka-shi, Osaka 559-8559 (JP)
(72) Inventor: YAMAKI, Masahiro, Osaka-shi, Osaka 559-8559 (JP); OHNO, Takuya, Osaka-shi, Osaka 559-8559 (JP); MORIYAMA, Tadahiro, Osaka-shi, Osaka 559-8559 (JP); ECHIGO, Takumi, Osaka-shi, Osaka 559-8559 (JP); SHIMAZAKI, Shingo, Osaka-shi, Osaka 559-8559 (JP)
(74) Representative: Grünecker Patent- und Rechtsanwälte PartG mbB
(86) International application number: PCT/JP2023/029490
(87) International publication number: WO 2024/075400

(57) **Abstract**

Provided is a technology of shortening a time required to heat a catalyst in a reaction tower. A generation device includes the reaction tower that generates a product gas by an exothermic reaction of a raw material gas in the catalyst and a raw material gas supply unit that heats the raw material gas and supplies the heated raw material gas to the reaction tower, and the reaction tower includes a reaction vessel that is filled with the catalyst and into which the raw material gas flows, a jacket portion that covers a portion of an outer periphery of the reaction vessel and in which a thermal medium flows, and a thermally insulating member covering another portion of the outer periphery of the reaction vessel.

## Description

### TECHNICAL FIELD

The present invention relates to a generation device and a generation method.

### BACKGROUND ART

For example, Patent Literature 1 discloses a technology related to a generation device and a generation method that generate a product gas by an exothermic reaction of a reactant in a gaseous state. For example, Patent Literature 2 discloses a technology related to a system that pre-heats a raw material gas to be supplied to a methanation reactor.

### CITATION LIST

### PATENT LITERATURE

Patent Literature 1: Japanese Patent No. 6984098
Patent Literature 2: Japanese Patent No. 6299347

### SUMMARY OF INVENTION

### TECHNICAL PROBLEM

A reaction tower to be used to generate a product gas is filled with a catalyst. By heating a thermal medium with a heater and circulating the heated thermal medium in the reaction tower, a temperature of the catalyst in the reaction tower is raised to a temperature required to start the generation of the product gas. To raise the temperature of the entire catalyst in the reaction tower by using the thermal medium, a large-scale heater is required, and also it takes time to heat the thermal medium.

The present invention has been achieved in view of the above circumstances, and an object thereof is to provide a technology capable of shortening a time required to heat a catalyst in a reaction tower.

### SOLUTION TO PROBLEM

To solve the problem described above, the present invention is a generation device including: a reaction tower that generates a product gas by an exothermic reaction of a raw material gas in a catalyst; and a raw material gas supply unit that heats the raw material gas and supplies the heated raw material gas to the reaction tower, the reaction tower including a reaction vessel that is filled with the catalyst and into which the raw material gas flows, a jacket portion that covers a portion of an outer periphery of the reaction vessel and in which a thermal medium flows, and a thermally insulating member covering another portion of the outer periphery of the reaction vessel.

In the generation device described above, the raw material gas is heated, and the heated raw material gas raises a temperature of the catalyst in the reaction tower and, accordingly, direct contact between the heated raw material gas and the catalyst shortens a time required for the temperature of the catalyst to reach a reaction start temperature. Therefore, it is possible to shorten a time required to heat the catalyst in the reaction tower. In addition, due to the thermally insulating member covering the portion of the outer periphery of the reaction vessel filled with the catalyst, the outer periphery of the reaction vessel is thermally insulated. This shortens the time required for the temperature of the catalyst in the reaction tower to reach the reaction start temperature during the supply the heated raw material gas to the reaction tower, and allows a reduction in the time required to heat the catalyst in the reaction tower.

It may also be possible that the thermally insulating member covers the outer periphery of the reaction vessel from a portion thereof into which the raw material gas flows to a predetermined portion thereof in a direction toward a center portion thereof. This thermally insulates the outer periphery of the reaction vessel in the vicinity of the portion thereof into which the raw material gas flows. When a heat spot is generated in the vicinity of the portion of the reaction vessel into which the raw material gas flows, by performing thermal insulation so as to cover the heat spot, it is possible to efficiently raise the temperature of the catalyst in the reaction tower.

It may also be possible that the generation device described above further includes: a delivery pump that sends the thermal medium to the reaction tower and that, after the supply of the raw material gas to the reaction tower is started, the delivery pump is activated to send the thermal medium to the reaction tower. This allows the thermal medium to be sent to the reaction tower after the start of the supply of the raw material gas to the reaction tower. For example, even when the thermal medium not sufficiently heated flows into the jacket portion of the reaction tower, the temperature of the catalyst is maintained by the portion of the reaction vessel covered with the thermally insulating member, and therefore it is possible to maintain the exothermic reaction in the reaction tower.

It may also be possible that the raw material gas supply unit determines a supply flow rate of the raw material gas to the reaction tower on the basis of an internal temperature of the reaction tower. This allows the raw material gas to be supplied at an appropriate supply flow rate to the reaction tower.

It may also be possible that, when a temperature of the catalyst in a portion of the reaction vessel that is covered with the thermally insulating member is not less than a predetermined temperature, the raw material gas supply unit gradually increases a supply flow rate of the raw material gas to the reaction tower. This accelerates the exothermic reaction of the raw material gas and increases an amount of the generated product gas.

It may also be possible that the generation device described above further includes: a combustion unit that burns the unreacted raw material gas delivered from the reaction tower and that at least one of the raw material gas supplied to the reaction tower and the thermal medium flowing into the jacket portion is heated by combustion heat of the unreacted raw material gas. This allows at least one of the raw material gas supplied to the reaction tower and the thermal medium flowing into the jacket portion to be heated by using combustion heat of the unreacted raw material gas, and allows the unreacted raw material gas to be effectively used.

It may also be possible that the generation device described above further includes: a second reaction tower that generates the product gas by an exothermic reaction of the raw material gas in a catalyst; and a circulation pump provided in a circulation path in which the thermal medium flows, that at least one of the product gas and the unreacted raw material gas that are delivered from the reaction tower is supplied into the second reaction tower, that, when the internal temperature of the reaction tower is not less than a predetermined temperature, the circulation pump is activated to circulate the thermal medium between the reaction tower and the second reaction tower, and that an internal capacity of the reaction tower is smaller than an internal capacity of the second reaction tower. Since the internal capacity of the reaction tower is smaller than the internal capacity of the second reaction tower, it is possible to shorten a time required to heat the catalyst in the reaction tower. By circulating the thermal medium between the reaction tower and the second reaction tower, it is possible to send the thermal medium heated by the exothermic reaction of the catalyst in the reaction tower to the second reaction tower.

It may also be possible that the generation device described above further includes: a switching unit that switches a destination of the raw material gas supplied from the raw material gas supply unit between the reaction tower and the second reaction tower and that the switching unit switches the destination of the raw material gas supplied from the raw material gas supply unit from the reaction tower to the second reaction tower. Thus, the raw material gas is supplied the second reaction tower, and the product gas is generated in the second reaction tower.

The present invention may also be a generation device including: a reaction tower that generates a product gas by an exothermic reaction of a raw material gas in a first catalyst; a boosting reaction tower that generates the product gas by an exothermic reaction of the raw material gas in a second catalyst; a raw material gas supply unit that heats the raw material gas and supplies the heated raw material gas to the boosting reaction tower; and a circulation pump provided in a circulation path in which a thermal medium flows, the boosting reaction tower including a reaction vessel that is filled with the second catalyst and into which the raw material gas flows, a jacket portion that covers a portion of an outer periphery of the reaction vessel and in which the thermal medium flows, and a thermally insulating member covering another portion of the outer periphery of the reaction vessel, at least one of the product gas and the unreacted raw material gas that are delivered from the boosting reaction tower being supplied into the reaction tower, when an internal temperature of the boosting reaction tower is not less than a predetermined temperature, the circulation pump being activated to circulate the thermal medium between the reaction tower and the boosting reaction tower, an internal capacity of the boosting reaction tower being smaller than an internal capacity of the reaction tower.

In the generation device described above, the raw material gas is heated, and the heated raw material gas raises a temperature of the second catalyst in the boosting reaction tower and, accordingly, a time required for the temperature of the second catalyst to reach a reaction start temperature is shortened. Therefore, it is possible to shorten a time required to heat the second catalyst in the boosting reaction tower. In addition, due to the thermally insulating member covering the portion of the outer periphery of the reaction vessel, the outer periphery of the reaction vessel is thermally insulated. This shortens the time required for the temperature of the second catalyst in the boosting reaction tower to reach the reaction start temperature during the supply of the heated raw material gas into the boosting reaction tower, and allows a reduction in the time required to heat the second catalyst in the boosting reaction tower. Since the internal capacity of the boosting reaction tower is smaller than the internal capacity of the second reaction tower, it is possible to shorten the time required to heat the second catalyst in the boosting reaction tower.

The present invention can also be understood from an aspect of a method. **In** other words, the present invention may also be a generation method of a product gas in a generation device including a reaction tower that generates the product gas by an exothermic reaction of a raw material gas in a catalyst, the generation method including the step of: heating the raw material gas and supplying the heated raw material gas to the reaction tower, the reaction tower including a reaction vessel that is filled with the catalyst and into which the raw material gas flows, a jacket portion that covers a portion of an outer periphery of the reaction vessel and in which a thermal medium flows, and a thermally insulating member covering another portion of the outer periphery of the reaction vessel.

According to the generation method described above, the raw material gas is heated, and the heated raw material gas raises a temperature of the catalyst in the reaction tower and, accordingly, a time required for the temperature of the catalyst to reach a reaction start temperature is shortened. Therefore, it is possible to shorten a time required to heat the catalyst in the reaction tower. **In** addition, due to the thermally insulating member covering the portion of the outer periphery of the reaction vessel, the outer periphery of the reaction vessel is thermally insulated. This shortens the time required for the temperature of the catalyst in the reaction tower to reach the reaction start temperature during the supply of the heated raw material gas to the reaction tower, and allows a reduction in the time required to heat the catalyst in the reaction tower.

Alternatively, the present invention may also be a generation method of a product gas in a generation device including: a reaction tower that generates the product gas by an exothermic reaction of a raw material gas in a first catalyst; a boosting reaction tower that generates the product gas by an exothermic reaction of the raw material gas in a second catalyst; and a circulation pump provided in a circulation path in which a thermal medium flows, the generation method including the steps of: heating the raw material gas and supplying the heated raw material gas to the boosting reaction tower; supplying at least one of the product gas and the unreacted raw material gas that are delivered from the boosting reaction tower into the reaction tower; and activating, when an internal temperature of the boosting reaction tower is not less than a predetermined temperature, the circular pump to circulate the thermal medium between the reaction tower and the boosting reaction tower, the boosting reaction tower including a reaction vessel that is filled with the second catalyst and into which the raw material gas flows, a jacket portion that covers a portion of an outer periphery of the reaction vessel and in which the thermal medium flows, and a thermally insulating member covering another portion of the outer periphery of the reaction vessel, an internal capacity of the boosting reaction tower being smaller than an internal capacity of the reaction tower.

In the generation method described above, the raw material gas is heated, and the heated raw material gas raises a temperature of the second catalyst in the boosting reaction tower and, accordingly, a time required for the temperature of the second catalyst to reach a reaction start temperature is shortened. Therefore, it is possible to shorten a time required to heat the second catalyst in the boosting reaction tower. In addition, due to the thermally insulating member covering the portion of the outer periphery of the reaction vessel, the outer periphery of the reaction vessel is thermally insulated. This shortens the time required for the temperature of the second catalyst in the boosting reaction tower to reach the reaction start temperature during the supply of the heated raw material gas into the boosting reaction tower, and allows a reduction in the time required to heat the second catalyst in the boosting reaction tower. Since the internal capacity of the boosting reaction tower is smaller than the internal capacity of the second reaction tower, it is possible to shorten the time required to heat the second catalyst in the boosting reaction tower.

### ADVANTAGEOUS EFFECTS OF INVENTION

It is possible to provide a technology capable of shortening a time required to heat a catalyst in a reaction tower.

### BRIEF DESCRIPTION OF DRAWINGS

[Fig. 1] Fig. 1 is a configuration diagram of a generation device according to a first embodiment.
[Fig. 2] Fig. 2A and Fig. 2B are diagrams illustrating an example of a configuration of a reaction tower.
[Fig. 3] Fig. 3 is a flow chart illustrating a flow of a procedure of operation of the generation device according to the first embodiment.
[Fig. 4] Fig. 4 is a diagram illustrating an example of a temperature distribution in the reaction tower after the start of the operation.
[Fig. 5] Fig. 5 is a configuration diagram of a generation device according to a second embodiment.
[Fig. 6] Fig. 6A is a diagram illustrating an example of a configuration of a boosting reaction tower, and Fig. 6B is a diagram illustrating an example of the configuration of the reaction tower.
[Fig. 7] Fig. 7 is a configuration diagram of the generation device according to the second embodiment.
[Fig. 8] Fig. 8 is a flow chart illustrating a flow of a procedure of operation of the generation device according to the second embodiment.

### DESCRIPTION OF EMBODIMENTS

A description will be given below of embodiments of the present invention. The following embodiments are examples of the embodiments of the present invention, and are not intended to limit the technical scope of the present invention to the following modes.

### <First Embodiment>

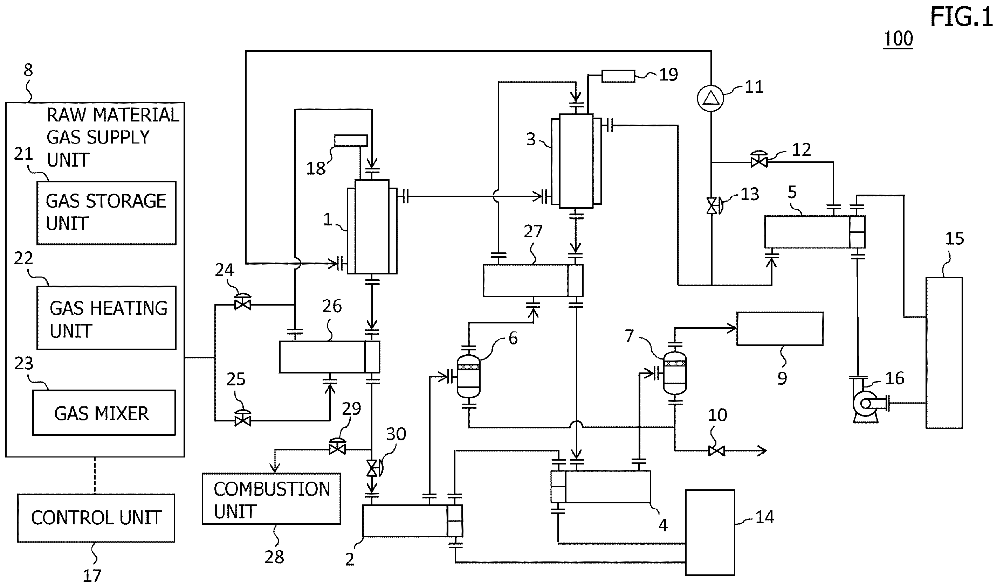

Fig. 1 is a configuration diagram of a generation device according to the first embodiment. A generation device 100 illustrated in Fig. 1 generates a methane gas serving as a product gas and water by an exothermic reaction between, e.g., gaseous hydrogen and carbon dioxide each serving as a raw material gas (reaction gas). The chemical reaction mentioned above is also a reversible reaction. The following is a chemical reaction formula representing the exothermic reaction mentioned above.

4H₂+CO₂→CH₄+2H₂O (1)

The generation device 100 includes a first-stage reaction tower (reactor) 1, a first-stage gas-cooling thermal exchanger 2, a second-stage reaction tower (reactor) 3, a second-stage gas-cooling thermal exchanger 4, a thermal-medium thermal exchanger 5, gas-liquid separators 6 and 7, and a raw material gas supply unit 8.

The reaction tower 1 generates the product gas by the exothermic reaction of the raw material gas in a catalyst. The raw material gas includes, e.g., hydrogen (H₂) and carbon dioxide (CO₂). The product gas is, e.g., the methane gas. The reaction tower 1 and the raw material gas supply unit 8 are connected via piping, and the raw material gas is supplied from the raw material gas supply unit 8 into the reaction tower 1. The reaction tower 1 also generates generated water by the exothermic reaction of the raw material gas in the catalyst. The reaction tower 1 and the gas-cooling thermal exchanger 2 are connected. In a path connecting the reaction tower 1 and the gas-cooling thermal exchanger 2, the piping, a valve, and the like are provided.

The gas-cooling thermal exchanger 2 condensates the generated water (water vapor) generated in the reaction tower 1. The gas-cooling thermal exchanger 2 and the gas-liquid separator 6 are connected. **In** a path connecting the gas-cooling thermal exchanger 2 and the gas-liquid separator 6, the piping, a valve, and the like are provided. The gas-liquid separator 6 separates the generated water (liquid) from the product gas and the unreacted raw material gas.

The reaction tower 3 and the gas-liquid separator 6 are connected. **In** a path connecting the reaction tower 3 and the gas-liquid separator 6, the piping, a valve, and the like are provided. The product gas and the unreacted raw material gas that are generated in the reaction tower 1 are sent to the reaction tower 3 via the gas-cooling thermal exchanger 2, the gas-liquid separator 6, and the like. The reaction tower 3 generates the product gas and the generated water by the exothermic reaction of the raw material gas in the catalyst. The generation of the product gas from the unreacted raw material gas in the reaction tower 3 allows the generation device 100 to generate a high-concentration product gas.

The reaction tower 3 and the gas-cooling thermal exchanger 4 are connected. **In a** path connecting the reaction tower 3 and the gas-cooling thermal exchanger 4, the piping, a valve, and the like are provided. The gas-cooling thermal exchanger 4 condensates the generated gas (water vapor) generated in the reaction tower 3. The gas-cooling thermal exchanger 4 and the gas-liquid separator 7 are connected. **In** a path connecting the gas-cooling thermal exchanger 4 and the gas-liquid separator 7, the piping, a valve, and the like are provided. The gas-liquid separator 7 separates the generated water (liquid) from the product gas and the unreacted raw material gas.

The generation device 100 includes a storage tank 9. From the gas-liquid separator 7 to the storage tank 9, the product gas is sent. The storage tank 9 stores the product gas. The gas-liquid separators 6 and 7 are provided with a water drain valve 10 for draining the generated water. The water drain valve 10 may be such that a valve is opened and closed by using a buoyant force of a floatation device, such as a drain trap, or that a water level is electrically detected to open and close a solenoid valve.

The reaction towers 1 and 3 are preliminarily filled with the catalyst. The catalyst may be any as long as the reaction formula (1) is accelerated thereby, and examples of the catalyst include a catalyst including a stabilized zirconia support having a stabilizing element dissolved therein and having a tetragonal and/or cubic crystalline structure and Ni supported by the stabilized zirconia support, in which the stabilizing element is at least one transition element selected from the group consisting of Mn, Fe, and Co.

Each of the reaction towers 1 and 3 has a jacket structure and, into and out of a jacket portion (shell) thereof, a thermal medium that exchanges heat with a heat generating portion in the reaction tower where the exothermic reaction occurs can flow. The thermal medium flows into the jacket portion of each of the reaction towers 1 and 3 to be circulated in each of the reaction towers 1 and 3. As the thermal medium, e.g., a heat transfer oil is used. The jacket portion of the reaction tower 1 and the jacket portion of the reaction tower 3 are connected via the piping in which the thermal medium flows. In the piping in which the thermal medium flows, a valve and the like are provided.

The jacket portion of the reaction tower 1 and the thermal-medium thermal exchanger 5 are connected by the piping in which the thermal medium flows. The jacket portion of the reaction tower 3 and the thermal-medium thermal exchanger 5 are connected by the piping in which the thermal medium flows. The thermal-medium thermal exchanger 5 cools the thermal medium that has passed through the reaction towers 1 and 3. The piping connecting the jacket portion of the reaction tower 1 and the thermal-medium thermal exchanger 5 is provided with a delivery pump 11 that sends the thermal medium to the reaction tower 1. In addition, the piping in which the thermal medium flows is provided with control valves 12 and 13. By opening and closing the control valves 12 and 13, the thermal medium that has passed through the reaction towers 1 and 3 can be sent to the reaction tower 1 via the thermal-medium thermal exchanger 5 or not via the thermal-medium thermal exchanger 5.

The generation device 100 includes a chiller 14. The chiller 14 cools cooling water (refrigerant) for condensing the generated water in the gas-cooling thermal exchangers 2 and 4. The gas-cooling thermal exchangers 2 and 4 and the chiller 14 are connected to each other by the piping in which the cooling water flows. The cooling water cooled by the chiller 14 returns to the chiller 14 via the gas-cooling thermal exchangers 2 and 4.

The generation device 100 includes a cooling tower 15 and a cooling-water circulation pump 16. The cooling tower 15 cools the cooling water that exchanges heat with the thermal medium in the thermal-medium thermal exchanger 5. For example, tap water supplied from outside of a system to the cooling tower 15 may also be used as the cooling water. The cooling-water circulation pump 16 circulates the cooling water supplied into the cooling tower 15 between the thermal-medium thermal exchanger 5 and the cooling tower 15.

The generation device 100 includes a control unit 17, a measurement sensor 18 that measures an internal temperature of the reaction tower 1, and a measurement sensor 19 that measures an internal temperature of the reaction tower 3. Measurement data resulting from the measurement by the measurement sensor 18 and measurement data resulting from the measurement by the measurement sensor 19 is sent to the raw material gas supply unit 8 and the control unit 17. Thus, the raw material gas supply unit 8 and the control unit 17 acquire the internal temperature of the reaction tower 1 and the internal temperature of the reaction tower 3.

The control unit 17 is a controller that controls entire operation of the generation device 100. The control unit 17 may be formed of a dedicated device or may also be formed of a versatile computer. The control unit 17 includes hardware resources such as a processor (CPU), a memory, a storage, and a communication I/F. The memory may be a RAM. The storage may be a nonvolatile storage device (such as, e.g., a ROM or a flash memory). Functions of the control unit 17 are implemented by expansion of a program stored in the storage in the memory and execution of the program by the processor. Note that a configuration of the control unit 17 is not limited thereto. For example, all or some of the functions may be formed of circuits such as an ASIC and a FPGA or, alternatively, all or some of the functions may also be performed by a cloud server or another device.

The control unit 17 controls the control valves 12 and 13. By opening of the control valve 12 and closing of the control valve 13, the thermal medium is sent to the reaction tower 1 via the thermal-medium thermal exchanger 5 to be cooled. As a result, the cooled thermal medium is sent to the reaction tower 1. By closing of the control valve 12 and opening of the control valve 13, the thermal medium is sent to the reaction tower 1 not via the thermal-medium thermal exchanger 5, and consequently the cooling of the thermal medium is stopped.

The raw material gas supply unit 8 heats the raw material gas and supplies the heated raw material gas to the reaction tower 1. The control unit 17 controls the raw material gas supply unit 8. As a result of control of operation of the raw material gas supply unit 8, the raw material gas supply unit 8 starts to supply the raw material gas to the reaction tower 1. Alternatively, the raw material gas supply unit 8 may also operate independently of the control unit 17.

The raw material gas supply unit 8 has a gas storage unit 21, a gas heating unit 22, and a gas mixer 23. The gas storage unit 21 stores the raw material gas. The gas heating unit 22 heats the raw material gas supplied from the raw material gas supply unit 8 to the reaction tower 1. For example, the gas heating unit 22 is a heater, a heat pump, an adiabatic compressor, or the like, but is not limited thereto. The gas heating unit 22 may heat the raw material gas stored in the gas storage unit 21 or may heat the raw material gas that enters the raw material gas supply unit 8 from outside. The gas heating unit 22 may also heat hydrogen included in the raw material gas. As a result of control of operation of the gas heating unit 22, the gas heating unit 22 heats the raw material gas or stops the heating of the raw material gas. By thus using the gas heating unit 22, the raw material gas is heated or the heating of the raw material gas is stopped. The gas mixer 23 uniformly mixes the raw material gas supplied to the reaction tower 1.

The generation device 100 includes control valves 24 and 25 and economizers 26 and 27. In a supply path for the raw material gas supplied from the raw material gas supply unit 8 to the reaction tower 1, the control valves 24 and 25 and the economizer 26 are provided. The control unit 17 controls the control valves 24 and 25. By closing of the control valve 24 and opening of the control valve 25, the raw material gas is supplied to the reaction tower 1 via the economizer 26. By opening of the control valve 24 and closing of the control valve 25, the raw material gas is supplied to the reaction tower 1 not via the economizer 26.

In the economizer 26, thermal exchange is performed between the raw material gas supplied to the reaction tower 1 and a delivered gas delivered from the reaction tower 1. The delivered gas is the product gas, the unreacted raw material gas, or a gas mixture of the product gas and the unreacted raw material gas. Accordingly, the delivered gas includes at least one of the product gas and the unreacted raw material gas. At a time when the supply of the raw material gas to the reaction tower 1 is started, the delivered gas has not been delivered from the reaction tower 1. Accordingly, it may also be possible that, immediately after the supply of the raw material gas to the reaction tower 1 is started, the raw material gas is supplied to the reaction tower 1 without being caused to pass through the economizer 26 and, at a time when the delivery of the delivered gas from the reaction tower 1 is started or after a lapse of a given time from the delivery of the delivered gas from the reaction tower 1, the raw material gas is caused to pass through the economizer 26 to be supplied to the reaction tower 1. Between the reaction tower 3 and the gas-liquid separator 6, the economizer 27 is provided. In the economizer 27, thermal exchange is performed between the delivered gas sent to the reaction tower 3 and the delivered gas delivered from the reaction tower 3.

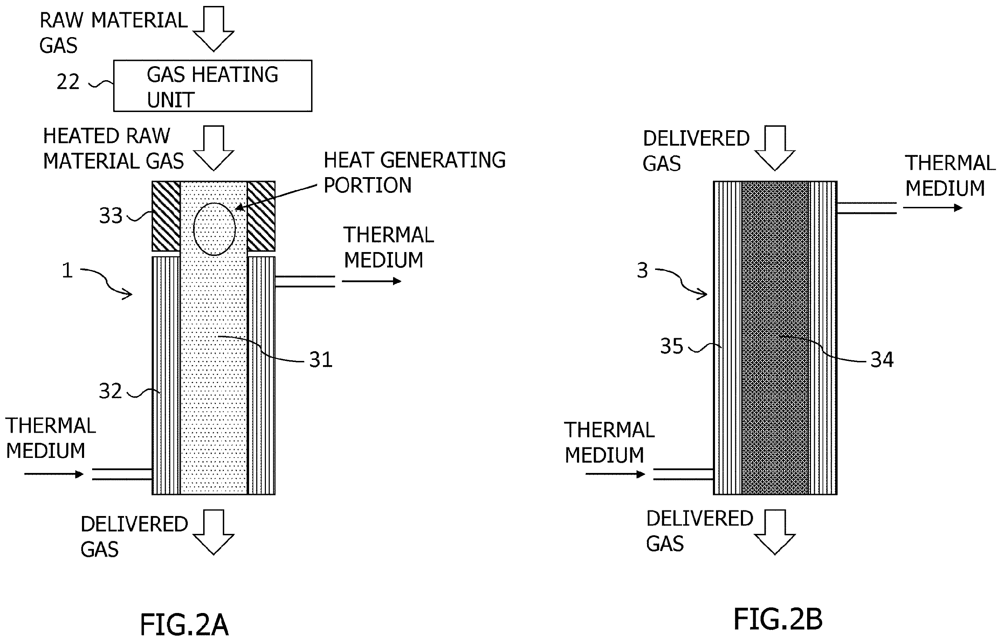

Fig. 2A is a diagram illustrating an example of a configuration of the reaction tower 1. Fig. 2A illustrates a cross section of the reaction tower 1. The reaction tower 1 includes a reaction vessel 31 filled with the catalyst, a jacket portion 32 provided so as to surround a lower portion of the reaction vessel 31, and a thermally insulating member 33 provided so as to surround an upper portion of the reaction vessel 31. The reaction vessel 31 has a structure in which a plurality of reaction tubes filled with the catalyst are provided to erect. From one end portion of the reaction vessel 31 toward another end portion of the reaction vessel 31, the plurality of reaction tubes extend. From an inlet end portion of the reaction vessel 31, the heated raw material gas flows into the reaction vessel 31 while, from an outlet end portion of the reaction vessel 31, the delivered gas flows out. When the delivery pump 11 is stopped and the thermal medium is not circulated between the reaction tower 1 and the reaction tower 3, the thermal medium stagnates in the jacket portion 32. When the delivery pump 11 is activated and the thermal medium is circulated between the reaction tower 1 and the reaction tower 3, the thermal medium flows in the jacket portion 32.

A material of the thermally insulating member 33 is not particularly limited as long as the material has a thermally insulating effect. As the thermally insulating member 33, a mantle heater may also be used. The mantle heater heats the catalyst in the reaction vessel 31, while having the thermally insulating effect. The thermally insulating member 33 is provided so as to surround the upper portion of the reaction vessel 31. For example, the thermally insulating member 33 covers a portion of an outer periphery of the reaction vessel 31 in the vicinity of the inlet end portion of the reaction vessel 31. Accordingly, the thermally insulating member 33 covers the outer periphery of the reaction vessel 31 from a portion (inlet end portion) thereof into which the raw material gas flows to a predetermined portion thereof in a direction toward a center portion thereof. The thermally insulating member 33 is not limited to the example described above, and may also cover at least a portion of the reaction vessel 31 in the vicinity of a heat spot. Alternatively, the thermally insulating member 33 may also cover at least a portion of the reaction vessel 31 on an upstream side of the heat spot. On a portion of the outer periphery of the reaction vessel 31 that is uncovered with the thermally insulating member 33, the jacket portion 32 is disposed. Thus, the jacket portion 32 covers a portion of the outer periphery of the reaction vessel 31, while the thermally insulating member 33 covers another portion of the outer periphery of the reaction vessel 31. Between the heat generating portion of the reaction vessel 31 and the thermal medium in the jacket portion 32, thermal exchange is performed. In other words, reaction heat generated in the reaction vessel 31 is transferred to the thermal medium in the jacket portion 32 to heat the thermal medium.

Fig. 2B is a diagram illustrating an example of a configuration of the reaction tower 3. Fig. 2B illustrates a cross section of the reaction tower 3. The reaction tower 3 includes a reaction vessel 34 filled with the catalyst and a jacket portion 35 provided so as to surround the reaction vessel 34. The reaction vessel 34 has a structure in which a plurality of reaction tubes filled with the catalyst are provided to erect. From one end portion of the reaction vessel 34 toward another end portion of the reaction vessel 34, the plurality of reaction tubes extend. From an inlet end portion of the reaction vessel 34, the delivered gas flows into the reaction vessel 34 while, from an outlet end portion of the reaction vessel 34, the delivered gas flows out.

When the delivery pump 11 is stopped and the thermal medium is not circulated between the reaction tower 1 and the reaction tower 3, the thermal medium stagnates in the jacket portion 35. When the delivery pump 11 is activated and the thermal medium is circulated between the reaction tower 1 and the reaction tower 3, the thermal medium flows in the jacket portion 35. Between the heat generating portion of the reaction vessel 34 and the thermal medium in the jacket portion 35, thermal exchange is performed. In other words, reaction heat generated in the reaction vessel 34 is transferred to the thermal medium in the jacket portion 35 to heat the thermal medium.

The measurement sensor 18 may also measure at least one of a temperature of the delivered gas delivered from the reaction tower 1 and a temperature of the thermal medium in the jacket portion 32 as the internal temperature of the reaction tower 1. The measurement sensor 19 may also measure at least one of a temperature of the delivered gas delivered from the reaction tower 3 and a temperature of the thermal medium in the jacket portion 35 as the internal temperature of the reaction tower 3.

### <Operation Procedure>

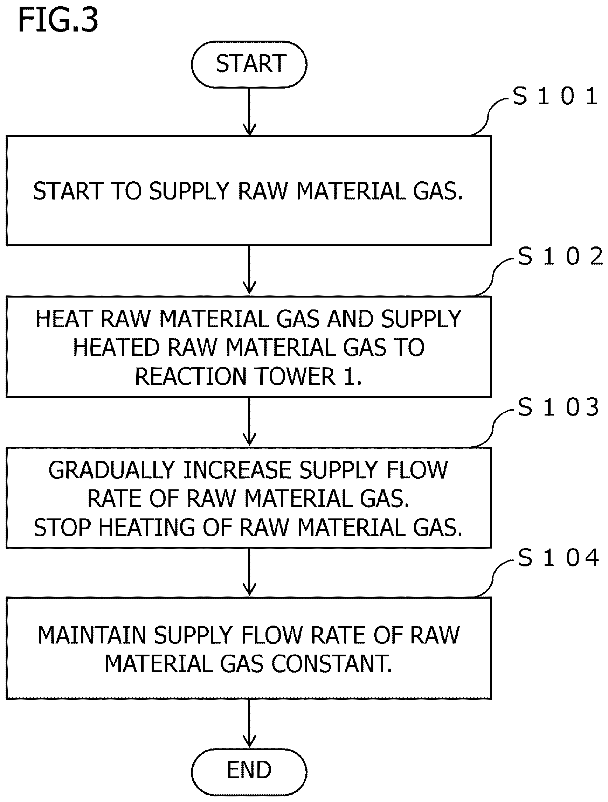

An operation procedure of the generation device 100 according to the first embodiment will be described. Fig. 3 is a flow chart illustrating a flow of the operation procedure of the generation device 100 according to the first embodiment. First, in Step S101, the raw material gas supply unit 8 is activated, and the raw material gas supply unit 8 starts to supply the raw material gas to the reaction tower 1. Then, in Step S102, the raw material gas supply unit 8 heats the raw material gas and supplies the heated raw material gas to the reaction tower 1. As a result of the supply of the heated raw material gas to the reaction tower 1, the heated raw material gas flows into the reaction vessel 31 from the inlet end portion thereof. The heated raw material gas flows into the reaction vessel 31 to raise a temperature of the catalyst in the portion (thermally insulated portion) of the reaction vessel 31 that is covered with the thermally insulating member 33. The temperature of the catalyst reaches a reaction start temperature and, by the exothermic reaction of the raw material gas that has flown into the reaction vessel 31, the product gas and the generated water are generated. In addition, the heated raw material gas heats the thermal medium in the reaction tower 1.

From the start of the supply of the raw material gas until the temperature of the catalyst reaches the reaction start temperature, a supply flow rate of the raw material gas is preferably reduced such that the exothermic reaction of the raw material gas is easily caused by the catalyst. Accordingly, the supply flow rate (first supply flow rate) of the raw material gas from the start of the supply of the raw material gas until the temperature of the catalyst reaches the reaction start temperature is lower than a rated supply flow rate (second supply flow rate) of the raw material gas when the product gas is generated. The raw material gas supply unit 8 or the control unit 17 may also determine the supply flow rate of the raw material gas to the reaction tower 1 on the basis of the internal temperature of the reaction tower 1. Alternatively, the raw material gas supply unit 8 or the control unit 17 may also determine the supply flow rate of the raw material gas to the reaction tower 1 on the basis of the temperature of the catalyst in the portion of the reaction vessel 31 that is covered with the thermally insulating member 33. The first supply flow rate may also be a flow rate at which the catalyst in the portion of the reaction vessel 31 that is covered with the thermally insulating member 33 remarkably accelerates the exothermic reaction of the raw material gas. By thus determining the supply flow rate of the raw material gas to the reaction tower 1 on the basis of the internal temperature of the reaction tower 1, the raw material gas can be supplied at an appropriate supply flow rate to the reaction tower 1.

In S103, when the temperature of the catalyst in the portion of the reaction vessel 31 that is covered with the thermally insulating member 33 is not less than a predetermined temperature (T1), the raw material gas supply unit 8 gradually increases the supply flow rate of the raw material gas and stops the heating of the raw material gas. The raw material gas supply unit 8 may also stop the heating of the raw material gas after performing the control to gradually increase the supply flow rate of the raw material gas. Alternatively, the raw material gas supply unit 8 may also perform control to gradually increase the supply flow rate of the raw material gas after stopping the heating of the raw material gas. The predetermined temperature (T1) is, e.g., 180°C, but is not limited to this temperature, and may also be determined on the basis of an experiment result, a simulation result, or the like.

When the temperature of the catalyst in the portion of the reaction vessel 31 that is covered with the thermally insulating member 33 reaches the predetermined temperature (T1), the exothermic reaction of the raw material gas is likely to occur, and an autonomous exothermic reaction in the reaction tower 1 is started. As a result, after the temperature of the catalyst in the portion of the reaction vessel 31 that is covered with the thermally insulating member 33 reaches the predetermined temperature (T1), the supply flow rate of the raw material gas is gradually increased to accelerate the exothermic reaction of the raw material gas and increase an amount of the generated product gas. In a case where the temperature of the catalyst in the portion of the reaction vessel 31 that is covered with the thermally insulating member 33 is not less than the predetermined temperature (T1), when the heated raw material gas is supplied to the reaction tower 1, the temperature of the catalyst in the reaction vessel 31 may be raised more than necessary. By stopping the heating of the raw material gas, the temperature of the catalyst in the reaction vessel 31 is prevented from being raised more than necessary. Even when the heating of the raw material gas is stopped, the catalyst in the portion of the reaction vessel 31 that is covered with the thermally insulating member 33 maintains the predetermined temperature (T1), and accordingly the exothermic reaction in the reaction tower 1 is continued. After the heating of the raw material gas is stopped, the thermal medium is heated by only the exothermic reaction in the reaction tower 1.

In Step S104, the raw material gas supply unit 8 increases the supply flow rate of the raw material gas until the supply flow rate of the raw material gas reaches the second supply flow rate (rated supply flow rate), and maintains the supply flow rate of the raw material gas constant. By maintaining the supply flow rate of the raw material gas at the rated supply flow rate, the product gas is stably delivered from the reaction tower 1.

The control unit 17 activates the delivery pump 11 with predetermined timing between Steps S101 and S104 in the flow chart in Fig. 3 to circulate the thermal medium between the reaction tower 1 and the reaction tower 3. The thermal medium passes through the reaction tower 1 by flowing in the jacket portion 32 of the reaction tower 1. The thermal medium that has passed through the reaction tower 1 is sent to the jacket portion 35 of the reaction tower 3 to pass through the reaction tower 3. The thermal medium passing through the reaction tower 3 has been heated by the reaction tower 1. The heated thermal medium passes through the reaction tower 3 to raise the internal temperature of the reaction tower 3. For example, even when the thermal medium that has not been sufficiently heated flows into the jacket portion 32 of the reaction tower 1, the temperature of the catalyst is maintained by the portion of the reaction vessel 31 that is covered with the thermally insulating member 33, and therefore the exothermic reaction in the reaction tower 1 can be maintained.

The control unit 17 may also compare at least one of the temperature of the delivered gas delivered from the reaction tower 1 and the temperature of the thermal medium in the jacket portion 32 with a threshold temperature, and determine timing with which the delivery pump 11 is to be activated on the basis of a result of the comparison.

The generation device 100 includes a combustion unit 28 and control valves 29 and 30. The combustion unit 28 burns the unreacted raw material gas delivered from the reaction tower 1. At least one of the raw material gas supplied to the reaction tower 1 and the thermal medium flowing into the jacket portion 32 is heated by combustion heat of the unreacted raw material gas. By using the combustion heat of the unreacted raw material gas, at least one of the raw material gas supplied to the reaction tower 1 and the thermal medium flowing into the jacket portion 32 can be heated, and the unreacted raw material gas can effectively be used. The control unit 17 controls the control valves 29 and 30. As a result of opening of the control valve 29 and closing of the control valve 30, the unreacted raw material gas delivered from the reaction tower 1 flows into the combustion unit 28. As a result of closing of the control valve 29 and opening of the control valve 30, the delivered gas delivered from the reaction tower 1 flows into the gas-cooling thermal exchanger 2.

From the start of the supply of the raw material gas to the reaction tower 1 until the predetermined timing, the temperature of the catalyst in the reaction tower 1 has not reached the reaction start temperature, and the unreacted raw material gas is delivered from the reaction tower 1. Accordingly, from the start of the supply of the raw material gas to the reaction tower 1 until the predetermined timing, it may also be possible to cause the unreacted raw material gas delivered from the reaction tower 1 to flow into the combustion unit 28. Alternatively, the generation device 100 may also include a thermal medium tank for storing the thermal medium.

In the generation device 100 according to the first embodiment, the raw material gas is heated, and the heated raw material gas raises the temperature of the catalyst in the reaction tower 1. Since specific heat of the raw material gas is small, the heating of the raw material gas is easier than when the thermal medium is heated. In the generation device 100 according to the first embodiment, the thermally insulating member 33 covers a predetermined portion of the outer periphery of the reaction vessel 31 to thermally insulate the outer periphery of the reaction vessel 31 in the vicinity of the inlet end portion thereof. This shortens the time required for the temperature of the catalyst in the reaction tower 1 to reach the reaction start temperature during the supply of the heated raw material gas to the reaction tower 1, and allows a reduction in the time required to heat the catalyst in the reaction tower 1. For example, when the heat transfer oil is used as the thermal medium, the heat transfer oil at a low temperature has a high viscosity and a poor fluidity, and accordingly it takes time for the heat transfer oil to circulate in the reaction tower. As a result, when the thermal medium is heated with a heater and the heated thermal medium is caused to pass through the reaction tower to raise the temperature of the catalyst, a time required for the temperature of the catalyst to reach the reaction start temperature is elongated.

In the generation device 100 according to the first embodiment, the raw material gas is heated, and the heated raw material gas raises the temperature of the catalyst in the reaction tower 1, which shortens the time required for the temperature of the catalyst to reach the reaction start temperature. Accordingly, it is possible to shorten the time required to heat the catalyst in the reaction tower 1 and shorten the time from the start of the supply of the raw material gas to the reaction tower 1 until the product gas is generated by the reaction tower 1. Thus, with the generation device 100 according to the first embodiment, it is possible to warm the catalyst in the reaction tower 1 without using a heater that heats the thermal medium and shorten the time until the operation of the reaction tower 1 is started. In addition, with the generation device 100 according to the first embodiment, it is possible to warm the catalyst in the reaction tower 3 without using a heater that heats the thermal medium and also shorten the time until the operation of the reaction tower 3 is started.

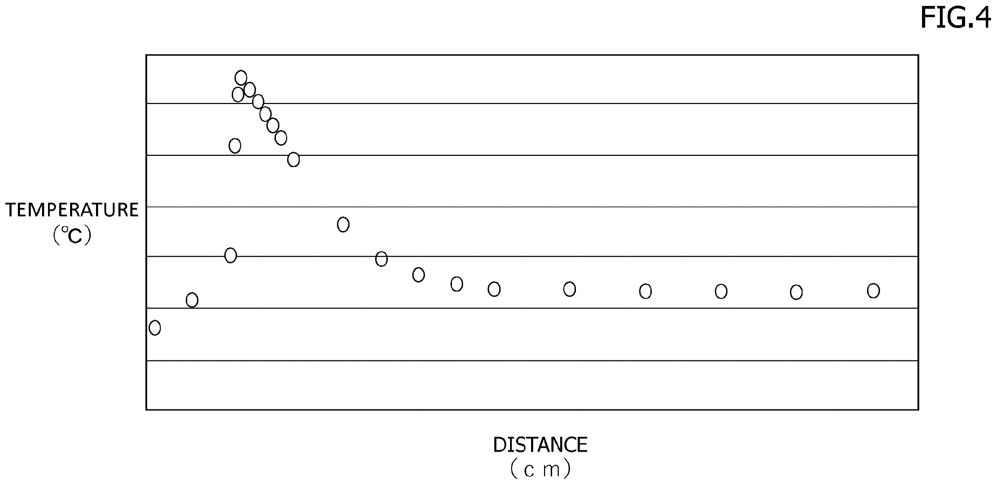

Fig. 4 is a diagram illustrating an example of a temperature distribution in a reaction tower after the start of operation thereof. In the reaction tower according to a reference example illustrated in Fig. 4, an entire outer periphery of a reaction vessel filled with a catalyst is covered with a jacket portion, and no thermally insulating member is provided. An ordinate axis in Fig. 4 represents an internal temperature (°C) of the reaction tower according to the reference example, while an abscissa axis in Fig. 4 represents a distance (cm) from an end portion (inlet end portion) of the reaction vessel according to the reference example for supplying a raw material gas. As illustrated in Fig. 4, in the vicinity of the inlet end portion of the reaction vessel according to the reference example, a heat spot is generated, and there is a peak temperature in the vicinity of the inlet end portion of the reaction vessel according to the reference example.

As illustrated in Fig. 4, a temperature in the vicinity of the inlet end portion of the reaction vessel according to the reference example has dropped toward an outlet end portion of the reaction vessel. This is because the raw material gas substantially reacts in the vicinity of the inlet end portion of the reaction vessel according to the reference example, and the raw material gas that reacts with a catalyst decreases toward the outlet end portion of the reaction vessel. Since the heat spot is generated in the vicinity of the inlet end portion of the reaction vessel 31, the thermally insulating member 33 preferably covers the outer periphery of the reaction vessel 31 from the inlet end portion thereof to a predetermined portion thereof in a direction toward a center portion thereof. This allows a temperature of the catalyst in the reaction tower 1 to be efficiently raised and also allows heat generated by an exothermic reaction in the reaction tower 1 to be efficiently transferred to the thermal medium. Alternatively, in the reaction tower 1, it may also be possible to cover the outer periphery of the reaction vessel 31 in a range from the inlet end portion thereof to a portion thereof where the heat spot is generated with the thermally insulating member 33. In the reaction tower 1, it may also be possible to cover the outer periphery of at least one portion of the reaction vessel 31 where the heat spot is generated with the thermally insulating member 33. In the reaction tower 1, it may also be possible to cover the outer periphery of at least one portion of the reaction vessel 31 in the vicinity of the portion thereof where the heat spot is generated with the thermally insulating member 33. In the reaction tower 1, it may also be possible to cover the outer periphery of at least one portion of the reaction vessel 31 on an upstream side of the portion thereof where the heat spot is generated with the thermally insulating member 33. The jacket portion 32 covers a portion of the outer periphery of the reaction vessel 31 and the thermally insulating member 33 covers another portion of the outer periphery of the reaction vessel 31 to allow a start-up time for the reaction tower 1 to be shortened, while allowing the exothermic reaction in the reaction tower 1 to be continued, while preventing the internal temperature of the reaction tower 1 from becoming higher than necessary. The start-up time for the reaction tower 1 is a time from the start of the supply of the raw material gas to the reaction tower 1 until the product gas is generated by the reaction tower 1.

For example, when the thermally insulating member 33 covers the entire outer periphery of the reaction vessel 31 or the thermally insulating member 33 covers the outer periphery of the reaction vessel 31 more than necessary, there is a concern that, due to an extreme temperature rise on a downstream side of the heat spot generated in the reaction vessel 31, the catalyst and the reaction tubes may be damaged. By covering the outer periphery of the reaction vessel 31 in the range from the inlet end portion thereof to the portion thereof where the heat spot is generated with the thermally insulating member 33, it is possible to prevent an extreme temperature rise on the downstream side of the heat spot generated in the reaction vessel 31. Due to the heat spot generated in the vicinity of the inlet end portion of the reaction vessel 31, the catalyst in the vicinity of the inlet end portion of the reaction vessel 31 may be damaged more seriously than the catalyst at another location. When the heat spot is generated in the vicinity of the inlet end portion of the reaction vessel 31, it may also be possible to set a frequency of replacement of the catalyst in the vicinity of the inlet end portion of the reaction vessel 31 high. Alternatively, by replacing only the catalyst in the vicinity of the inlet end portion of the reaction vessel 31, catalyst replacement cost can be reduced compared with that when the entire catalyst in the reaction vessel 31 is replaced.

### <Second Embodiment>

A description will be given of the second embodiment. In the second embodiment, the same components as those in the first embodiment are designated by the same reference signs as those in the first embodiment, and a description thereof is omitted appropriately. It may also be possible to appropriately combine the generation devices 100 according to the first and second embodiments with each other.

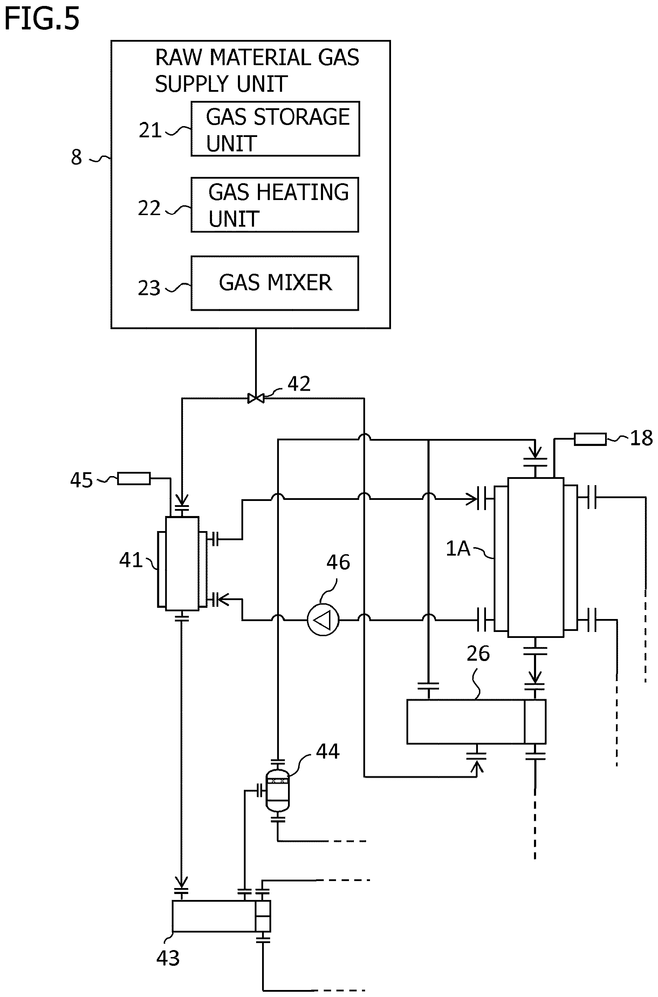

Fig. 5 is a configuration diagram of the generation device 100 according to the second embodiment. Fig. 5 illustrates a portion of the generation device 100. As compared with the generation device 100 according to the first embodiment, the generation device 100 according to the second embodiment includes a reaction tower 1A instead of the reaction tower 1. As also compared with the generation device 100 according to the first embodiment, the generation device 100 according to the second embodiment further includes a boosting reaction tower 41, a switching unit 42, a gas-cooling thermal exchanger 43, a gas-liquid separator 44, a measurement sensor 45, and a circulation pump 46.

The boosting reaction tower 41 generates the product gas by the exothermic reaction of the raw material gas in the catalyst. The raw material gas supply unit 8 and the boosting reaction tower 41 are connected by piping, and the switching unit 42 is provided midway in the piping connecting the raw material gas supply unit 8 and the boosting reaction tower 41. The switching unit 42 is, e.g., a three-way valve. The switching unit 42 switches a destination of the raw material gas supplied from the raw material gas supply unit 8 between the reaction tower 1A and the boosting reaction tower 41. The control unit 17 may also control the switching by the switching unit 42. When the destination of the raw material gas supplied from the raw material gas supply unit 8 is switched from the boosting reaction tower 41 to the reaction tower 1A, the raw material gas is supplied from the raw material gas supply unit 8 into the reaction tower 1A. When the destination of the raw material gas supplied from the raw material gas supply unit 8 is switched from the reaction tower 1A to the boosting reaction tower 41, the raw material gas is supplied from the raw material gas supply unit 8 to the boosting reaction tower 41.

The boosting reaction tower 41 generates the product gas and the generated water by the exothermic reaction of the raw material gas in the catalyst. The delivered gas delivered from the boosting reaction tower 41 is sent to the reaction tower 1A via the gas-cooling thermal exchanger 43 and the gas-liquid separator 44 to be supplied into the reaction tower 1A. The reaction tower 1A generates the product gas and the generated water by the exothermic reaction of the raw material gas in the catalyst.

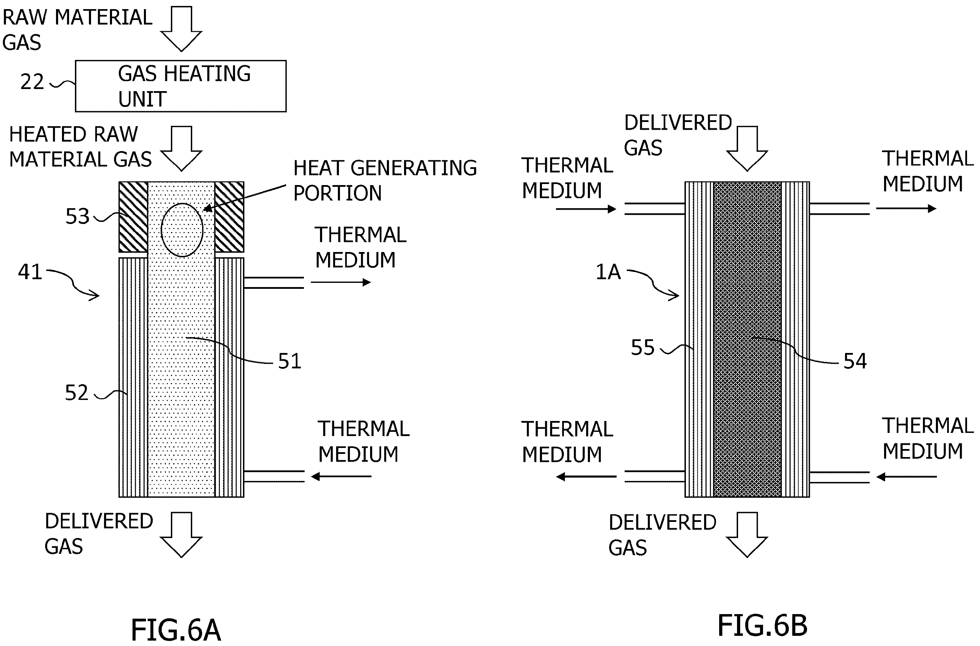

Fig. 6A is a diagram illustrating an example of a configuration of the boosting reaction tower 41. Fig. 6A illustrates a cross section of the boosting reaction tower 41. The boosting reaction tower 41 includes a reaction vessel 51 filled with the catalyst, a jacket portion 52 provided so as to surround a lower portion of the reaction vessel 51, and a thermally insulating member 53 provided so as to surround an upper portion of the reaction vessel 51. From an inlet end portion of the reaction vessel 51, the heated raw material gas flows into the reaction vessel 51 and, from an outlet end portion of the reaction vessel 51, the delivered gas flows out. When the circulation pump 46 is stopped and the thermal medium is not circulated between the boosting reaction tower 41 and the reaction tower 1A, the thermal medium stagnates in the jacket portion 52. When the circulation pump 46 is activated and the thermal medium is circulated between the boosting reaction tower 41 and the reaction tower 1A, the thermal medium flows in the jacket portion 52.

Between a heat generating portion of the reaction vessel 51 and the thermal medium in the jacket portion 52, thermal exchange is performed. In other words, reaction heat generated in the reaction vessel 51 is transferred to the thermal medium in the jacket portion 52 to heat the thermal medium. A configuration of the boosting reaction tower 41 is the same as that of the reaction tower 1 according to the first embodiment, but an internal capacity of the boosting reaction tower 41 is smaller than the internal capacity of the reaction tower 1. In addition, the internal capacity of the boosting reaction tower 41 is smaller than an internal capacity of the reaction tower 1A and also smaller than the internal capacity of the reaction tower 3.

Fig. 6B is a diagram illustrating an example of a configuration of the reaction tower 1A. Fig. 6B illustrates a cross section of the reaction tower 1A. The reaction tower 1A includes a reaction vessel 54 filled with the catalyst and a jacket portion 55 provided so as to surround the reaction vessel 54. From an inlet end portion of the reaction vessel 54, the delivered gas flows into the reaction vessel 54 and, from an outlet end portion of the reaction vessel 54, the delivered gas flows out.

When the delivery pump 11 and the circulation pump 46 are stopped, the thermal medium is not circulated between the boosting reaction tower 41 and the reaction tower 1A, and the thermal medium is not circulated between the reaction tower 1A and the reaction tower 3, the thermal medium stagnates in the jacket portion 55. When the circulation pump 46 is activated and the thermal medium is circulated between the boosting reaction tower 41 and the reaction tower 1A, the thermal medium flows in the jacket portion 55. Alternatively, when the delivery pump 11 is activated and the thermal medium is circulated between the reaction tower 1A and the reaction tower 3, the thermal medium flows in the jacket portion 55. Between a heat generating portion of the reaction vessel 54 and the thermal medium in the jacket portion 55, thermal exchange is performed. In other words, reaction heat generated in the reaction vessel 54 is transferred to the thermal medium in the jacket portion 55 to heat the thermal medium.

Respective configurations of the reaction vessel 51, the jacket portion 52, and the thermally insulating member 53 of the boosting reaction tower 41 are the same as respective configurations of the reaction vessel 31, the jacket portion 32, and the thermally insulating member 33 of the reaction tower 1 according to the first embodiment. Respective configurations of the reaction vessel 54 and the jacket portion 55 of the reaction tower 1A are the same as respective configurations of the reaction vessel 34 and the jacket portion 35 of the reaction tower 3 according to the first embodiment. However, the jacket portion 52 of the boosting reaction tower 41 and the jacket portion 55 of the reaction tower 1A are connected to allow the thermal medium to be circulated between the boosting reaction tower 41 and the reaction tower 1A. Meanwhile, the jacket portion 55 of the boosting reaction tower 1A and the jacket portion 35 of the reaction tower 3 are connected to allow the thermal medium to be circulated between the reaction tower 1A and the reaction tower 3.

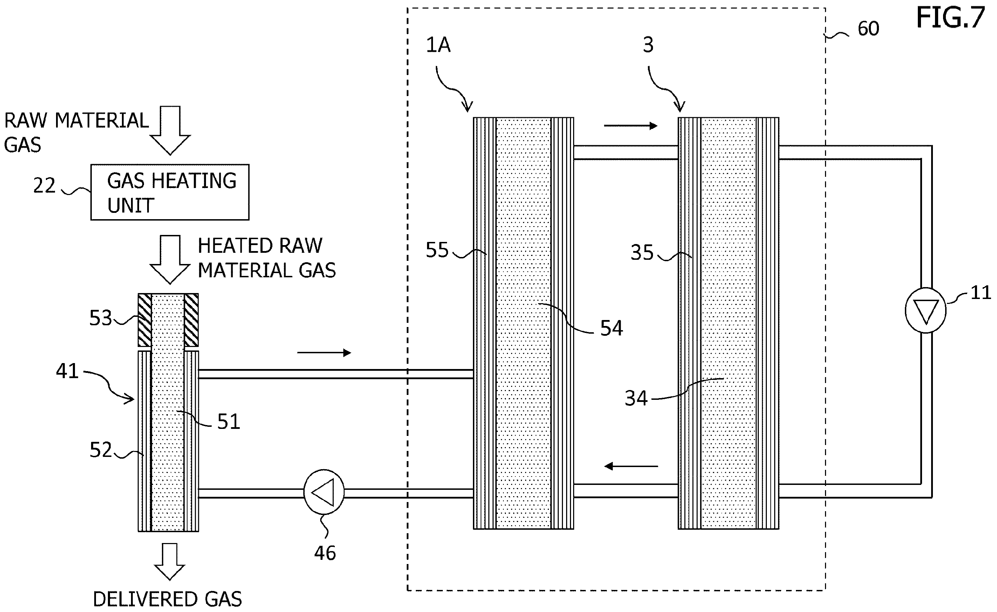

Fig. 7 is a configuration diagram of the generation device 100 according to the second embodiment. Fig. 7 illustrates a portion of the generation device 100. Fig. 7 illustrates the boosting reaction tower 41 and a main circuit reaction group 60 including the reaction tower 1A and the reaction tower 3. In the main circuit reaction group 60, by operation of the delivery pump 11 provided in a circulation path in which the thermal medium flows, the thermal medium is circulated between the reaction tower 1A and the reaction tower 3. The circulation pump 46 is disposed between the boosting reaction tower 41 and the reaction tower 1A to be provided in the circulation path in which the thermal medium flows. By operation of the circulation pump 46, the thermal medium is circulated between the boosting reaction tower 41 and the reaction tower 1A.

The measurement sensor 18 measures an internal temperature of the reaction tower 1A, while the measurement sensor 45 measures an internal temperature of the boosting reaction tower 41. Measurement data obtained by the measurement performed by the measurement sensor 18 and measurement data obtained by the measurement performed by the measurement sensor 45 is sent to the raw material gas supply unit 8 and the control unit 17. Thus, the raw material gas supply unit 8 and the control unit 17 acquire an internal temperature of the reaction tower 1A and the internal temperature of the boosting reaction tower 41.

The measurement sensor 18 may also measure at least one of a temperature of the delivered gas delivered from the reaction tower 1A and a temperature of the thermal medium in the jacket portion 55 as the internal temperature of the reaction tower 1A. The measurement sensor 45 may also measure at least one of a temperature of the delivered gas delivered from the boosting reaction tower 41 and a temperature of the thermal medium in the jacket portion 52 as the internal temperature of the boosting reaction tower 41.

### <Operation Procedure>

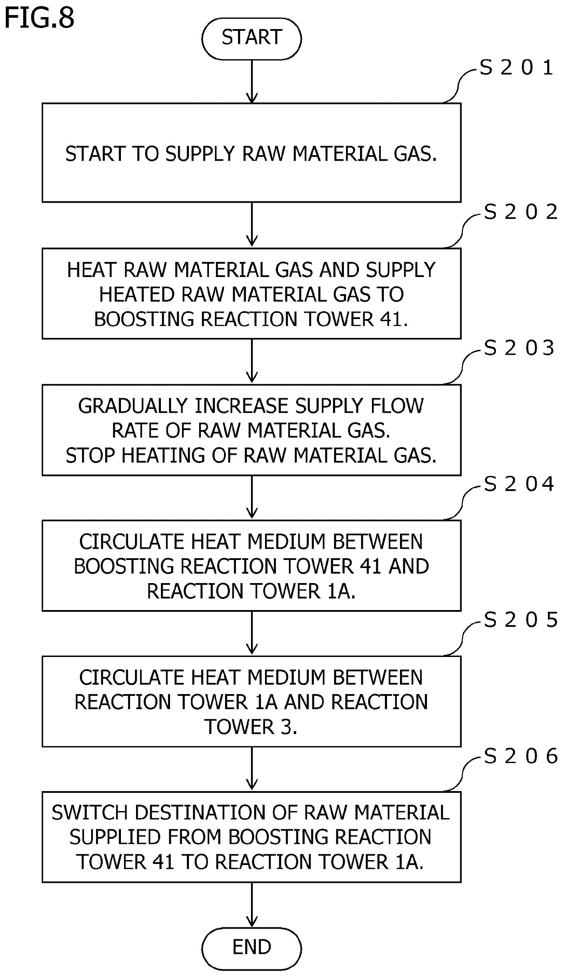

An operation procedure of the generation device 100 according to the second embodiment will be described. Fig. 8 is a flow chart illustrating a flow of the operation procedure of the generation device 100 according to the second embodiment. First, in Step S201, the raw material gas supply unit 8 is activated, and the raw material gas supply unit 8 starts to supply the raw material gas to the boosting reaction tower 41. Then, in Step S202, the raw material gas supply unit 8 heats the raw material gas and supplies the heated raw material gas to the boosting reaction tower 41. As a result of the supply of the heated raw material gas to the boosting reaction tower 41, the heated raw material gas flows into the reaction vessel 51 from the inlet end portion thereof. The heated raw material gas flows into the reaction vessel 51 to raise the temperature of the catalyst in the portion of the reaction vessel 51 that is covered with the thermally insulating member 53. The temperature of the catalyst reaches the reaction start temperature and, by the exothermic reaction of the raw material gas that has flown into the reaction vessel 51, the product gas and the generated water are generated.

The supply flow rate (first supply flow rate) of the raw material gas from the start of the supply of the raw material gas until the temperature of the catalyst reaches the reaction start temperature is lower than the rated supply flow rate (second supply flow rate) of the raw material gas when the product gas is generated. The raw material gas supply unit 8 or the control unit 17 may also determine the supply flow rate of the raw material gas to the boosting reaction tower 41 on the basis of the internal temperature of the boosting reaction tower 41. Alternatively, the raw material gas supply unit 8 or the control unit 17 may also determine the supply flow rate of the raw material gas to the boosting reaction tower 41 on the basis of the temperature of the catalyst in the portion of the reaction vessel 51 that is covered with the thermally insulating member 53. Preferably, the first supply flow rate is a flow rate at which the catalyst in the portion of the reaction vessel 51 that is covered with the thermally insulating member 53 remarkably accelerates the exothermic reaction of the raw material gas.

In S203, when the temperature of the catalyst in the portion of the reaction vessel 51 that is covered with the thermally insulating member 53 is not less than a predetermined temperature (T2), the raw material gas supply unit 8 gradually increases the supply flow rate of the raw material gas and stops the heating of the raw material gas. The raw material gas supply unit 8 may also stop the heating of the raw material gas after performing the control to gradually increase the supply flow rate of the raw material gas. Alternatively, the raw material gas supply unit 8 may also perform the control to gradually increase the supply flow rate of the raw material gas after stopping the heating of the raw material gas. The predetermined temperature (T2) is, e.g., 180°C, but is not limited to this temperature, and may also be determined on the basis of an experiment result, a simulation result, or the like.

When the temperature of the catalyst in the portion of the reaction vessel 51 that is covered with the thermally insulating member 53 reaches the predetermined temperature (T2), an autonomous exothermic reaction in the boosting reaction tower 41 is started. As a result, after the temperature of the catalyst in the portion of the reaction vessel 51 that is covered with the thermally insulating member 53 reaches the predetermined temperature (T2), the supply flow rate of the raw material gas is gradually increased to increase the amount of the generated product gas. In a case where the temperature of the catalyst in the portion of the reaction vessel 51 that is covered with the thermally insulating member 53 is not less than the predetermined temperature (T2), when the heated raw material gas is supplied to the boosting reaction tower 41, the temperature of the catalyst in the reaction vessel 51 may be raised more than necessary. By stopping the heating of the raw material gas, the temperature of the catalyst in the reaction vessel 51 is prevented from being raised more than necessary.

In Step S204, when a temperature of the delivered gas delivered from the boosting reaction tower 41 is not less than a predetermined temperature (T3), the control unit 17 activates the circulation pump 46 to circulate the thermal medium between the boosting reaction tower 41 and the reaction tower 1A. The thermal medium passes through the boosting reaction tower 41 by flowing in the jacket portion 52 of the boosting reaction tower 41. The thermal medium that has passed through the boosting reaction tower 41 is sent to the jacket portion 55 of the reaction tower 1A to pass through the reaction tower 1A. The thermal medium passing through the reaction tower 1A has been heated by the boosting reaction tower 41. The heated thermal medium passes through the reaction tower 1A to raise the internal temperature of the reaction tower 1A. The predetermined temperature (T3) is, e.g., 180°C, but is not limited to this temperature, and may also be determined on the basis of an experiment result, a simulation result, or the like.

In Step S204, when the temperature of the thermal medium in the jacket portion 52 is not less than a predetermined temperature (T4), the control unit 17 may also activate the circulation pump 46 to circulate the thermal medium between the boosting reaction tower 41 and the reaction tower 1A. The predetermined temperature (T4) is, e.g., 180°C, but is not limited to this temperature, and may also be determined on the basis of an experiment result, a simulation result, or the like.

In Step S205, the control unit 17 activates the circulation pump 46 to circulate the thermal medium between the reaction tower 1A and the reaction tower 3. The thermal medium that has passed through the reaction tower 1A is sent to the jacket portion 35 of the reaction tower 3 to pass through the reaction tower 3. The thermal medium passing through the reaction tower 3 has been heated by the reaction tower 1A. The heated thermal medium passes through the reaction tower 3 to raise the internal temperature of the reaction tower 3.

In Step S206, the switching unit 42 switches the destination of the raw material gas supplied from the raw material gas supply unit 8 from the boosting reaction tower 41 to the reaction tower 1A. As a result, the raw material gas is supplied from the raw material gas supply unit 8 to the reaction tower 1A. Since the internal temperature of the reaction tower 1A has been raised, a time required for the temperature of the catalyst to reach the reaction start temperature is shortened. Accordingly, it is possible to shorten the time required to heat the catalyst in the reaction tower 1A and shorten the time from the start of the supply of the raw material gas to the reaction tower 1A until the product gas is generated by the reaction tower 1A.

In the flow chart in Fig. 8, timing with which processing in Step S204 is performed may be the same as or different from timing with which processing in Step S205 is performed. In the flow chart in Fig. 8, it may also be possible that, after processing in Step S206 is performed, the processing in Step S204 and S205 is performed. It may also be possible that, when the temperature of the delivered gas delivered from the boosting reaction tower 41 is not less than the predetermined temperature (T3) in Step S206, the destination of the raw material gas supplied from the raw material gas supply unit 8 is switched from the boosting reaction tower 41 to the reaction tower 1A. It may also be possible that, when the temperature of the thermal medium in the jacket portion 52 is not less than the predetermined temperature (T4) in Step S206, the destination of the raw material gas supplied from the raw material gas supply unit 8 is switched from the boosting reaction tower 41 to the reaction tower 1A.

In the generation device 100 according to the second embodiment, the raw material gas is heated, and the heated raw material gas raises the temperature of the catalyst in the boosting reaction tower 41. Since specific heat of the raw material gas is small, the heating of the raw material gas is easier than when the thermal medium is heated. In the generation device 100 according to the second embodiment, the thermally insulating member 53 covers a predetermined portion of the outer periphery of the reaction vessel 51 to thermally insulate the outer periphery of the reaction vessel 51 in the vicinity of the inlet end portion thereof. This shortens the time required for the temperature of the catalyst in the boosting reaction tower 41 to reach the reaction start temperature during the supply of the heated raw material gas to the boosting reaction tower 41, and allows a reduction in the time required to heat the catalyst in the boosting reaction tower 41. For example, when the heat transfer oil is used as the thermal medium, the heat transfer oil at a low temperature has a high viscosity and a poor fluidity, and accordingly it takes time for the heat transfer oil to circulate in the reaction tower. As a result, when the thermal medium is heated with a heater and the heated thermal medium is caused to pass through the reaction tower to raise the temperature of the catalyst, the time required for the temperature of the catalyst to reach the reaction start temperature is elongated.

In the generation device 100 according to the second embodiment, the raw material gas is heated, and the heated raw material gas raises the temperature of the catalyst in the boosting reaction tower 41, which shortens the time required for the temperature of the catalyst to reach the reaction start temperature. Accordingly, it is possible to shorten the time required to heat the catalyst in the boosting reaction tower 41 and shorten the time from the start of the supply of the raw material gas to the boosting reaction tower 41 until the product gas is generated by the boosting reaction tower 41. Thus, with the generation device 100 according to the second embodiment, it is possible to warm the catalyst in the boosting reaction tower 41 without using a heater that heats the thermal medium and shorten the time until the operation of the boosting reaction tower 41 is started. In addition, with the generation device 100 according to the second embodiment, it is possible to warm the catalyst in the reaction tower 1A and the catalyst in the reaction tower 3 without using a heater that heats the thermal medium and also shorten the time until the operation of the reaction tower 1A and the reaction tower 3 is started.

The internal capacity of the boosting reaction tower 41 is smaller than the internal capacity of the reaction tower 1A, and accordingly it is possible to shorten the time required to heat the catalyst in the boosting reaction tower 41. By circulating thermal medium between the boosting reaction tower 41 and the reaction tower 1A, it is possible to send the thermal medium heated by the exothermic reaction of the catalyst in the boosting reaction tower 41 to the reaction tower 1A.

When the operation of the generation device 100 is started, a heated fuel gas is supplied to the boosting reaction tower 41, and the heating of the fuel gas is stopped with predetermined timing. In addition, the destination of the fuel gas to be supplied is switched from the boosting reaction tower 41 to the reaction tower 1A with predetermined timing. This prevents catalyst poisoning and can reduce a frequency of replacement of the catalyst.

Due to the heat spot generated in the vicinity of the inlet end portion of the reaction vessel 51, the catalyst in the vicinity of the inlet end portion of the reaction vessel 51 may be damaged more seriously than the catalyst at another location. When the heat spot is generated in the vicinity of the inlet end portion of the reaction vessel 51, a frequency of replacement of the catalyst in the vicinity of the inlet end portion of the reaction vessel 51 may also be increased. By replacing only the catalyst in the vicinity of the inlet end portion of the reaction vessel 51, the catalyst replacement cost can be reduced to be lower than when the entire catalyst in the reaction vessel 51 is replaced.

Each processing described above may also be considered as a production method of the product gas in the generation device 100, an operation method of the generation device 100, or the like. Each processing described above may also be considered as a generation system or an operation system having at least part of each of the processing and functions described above. Note that each of the means and processing described above can be combined with each other as much as possible to constitute the present invention.

### REFERENCE SIGNS LIST

1, 1A, 3 Reaction tower; 2, 4 Gas-cooling thermal exchanger; 5 Thermal-medium thermal exchanger; 6, 7 Gas-liquid separator; 8 Raw material gas supply unit; 11 Delivery pump; 17 Control unit; 18, 19, 45 Measurement sensor; 28 Combustion unit; 31, 34, 51, 54 Reaction vessel; 32, 35, 52, 55 Jacket portion; 33, 53 Thermally insulating member; 41 Boosting reaction tower; 42 Switching unit; 46 Circulation pump; 100 Generation device;

## Claims

1. A generation device comprising:
a reaction tower that generates a product gas by an exothermic reaction of a raw material gas in a catalyst; and
a raw material gas supply unit that heats the raw material gas and supplies the heated raw material gas to the reaction tower,
the reaction tower including a reaction vessel that is filled with the catalyst and into which the raw material gas flows, a jacket portion that covers a portion of an outer periphery of the reaction vessel and in which a thermal medium flows, and a thermally insulating member covering another portion of the outer periphery of the reaction vessel.

2. The generation device according to claim 1, wherein the thermally insulating member covers the outer periphery of the reaction vessel from a portion thereof into which the raw material gas flows to a predetermined portion thereof in a direction toward a center portion thereof.

3. The generation device according to claim 1, further comprising:
a delivery pump that sends the thermal medium to the reaction tower, wherein,
after the supply of the raw material gas to the reaction tower is started, the delivery pump is activated to send the thermal medium to the reaction tower.

4. The generation device according to claim 1, wherein the raw material gas supply unit determines a supply flow rate of the raw material gas to the reaction tower on the basis of an internal temperature of the reaction tower.

5. The generation device according to claim 1, wherein, when a temperature of the catalyst in a portion of the reaction vessel that is covered with the thermally insulating member is not less than a predetermined temperature, the raw material gas supply unit gradually increases a supply flow rate of the raw material gas to the reaction tower.

6. The generation device according to claim 1, further comprising:
a combustion unit that burns the unreacted raw material gas delivered from the reaction tower, wherein
at least one of the raw material gas supplied to the reaction tower and the thermal medium flowing into the jacket portion is heated by combustion heat of the unreacted raw material gas.

7. The generation device according to any one of claims 1 to 6, further comprising:
a second reaction tower that generates the product gas by an exothermic reaction of the raw material gas in a second catalyst; and
a circulation pump provided in a circulation path in which the thermal medium flows, wherein
at least one of the product gas and the unreacted raw material gas that are delivered from the reaction tower is supplied into the second reaction tower,
when the internal temperature of the reaction tower is not less than a predetermined temperature, the circulation pump is activated to circulate the thermal medium between the reaction tower and the second reaction tower, and
an internal capacity of the reaction tower is smaller than an internal capacity of the second reaction tower.

8. The generation device according to claim 7, further comprising:
a switching unit that switches a destination of the raw material gas supplied from the raw material gas supply unit between the reaction tower and the second reaction tower, wherein
the switching unit switches the destination of the raw material gas supplied from the raw material gas supply unit from the reaction tower to the second reaction tower.

9. A generation device comprising:
a reaction tower that generates a product gas by an exothermic reaction of a raw material gas in a first catalyst;
a boosting reaction tower that generates the product gas by an exothermic reaction of the raw material gas in a second catalyst;
a raw material gas supply unit that heats the raw material gas and supplies the heated raw material gas to the boosting reaction tower; and
a circulation pump provided in a circulation path in which a thermal medium flows,
the boosting reaction tower including a reaction vessel that is filled with the second catalyst and into which the raw material gas flows, a jacket portion that covers a portion of an outer periphery of the reaction vessel and in which the thermal medium flows, and a thermally insulating member covering another portion of the outer periphery of the reaction vessel,
at least one of the product gas and the unreacted raw material gas that are delivered from the boosting reaction tower being supplied into the reaction tower,
when an internal temperature of the boosting reaction tower is not less than a predetermined temperature, the circulation pump being activated to circulate the thermal medium between the reaction tower and the boosting reaction tower,
an internal capacity of the boosting reaction tower being smaller than an internal capacity of the reaction tower.

10. A generation method of a product gas in a generation device including a reaction tower that generates the product gas by an exothermic reaction of a raw material gas in a catalyst, the generation method comprising the step of:
heating the raw material gas and supplying the heated raw material gas to the reaction tower,
the reaction tower including a reaction vessel that is filled with the catalyst and into which the raw material gas flows, a jacket portion that covers a portion of an outer periphery of the reaction vessel and in which a thermal medium flows, and a thermally insulating member covering another portion of the outer periphery of the reaction vessel.

11. A generation method of a product gas in a generation device including:
a reaction tower that generates the product gas by an exothermic reaction of a raw material gas in a first catalyst;
a boosting reaction tower that generates the product gas by an exothermic reaction of the raw material gas in a second catalyst; and
a circulation pump provided in a circulation path in which a thermal medium flows, the generation method comprising the steps of:
heating the raw material gas and supplying the heated raw material gas to the boosting reaction tower;
supplying at least one of the product gas and the unreacted raw material gas that are delivered from the boosting reaction tower into the reaction tower; and
activating, when an internal temperature of the boosting reaction tower is not less than a predetermined temperature, the circular pump to circulate the thermal medium between the reaction tower and the boosting reaction tower,
the boosting reaction tower including a reaction vessel that is filled with the second catalyst and into which the raw material gas flows, a jacket portion that covers a portion of an outer periphery of the reaction vessel and in which the thermal medium flows, and a thermally insulating member covering another portion of the outer periphery of the reaction vessel,
an internal capacity of the boosting reaction tower being smaller than an internal capacity of the reaction tower.
